Europäisches Patentamt

European Patent Office

Office européen des brevets

(11) Veröffentlichungsnummer: **0 671 405 A1**

(12) **EUROPÄISCHE PATENTANMELDUNG**

(21) Anmeldenummer: **95100155.1**

(22) Anmeldetag: **07.01.95**

(51) Int. Cl.6: **C07H 7/027**

(30) Priorität: **18.01.94 CH 154/94**

(43) Veröffentlichungstag der Anmeldung:
**13.09.95 Patentblatt 95/37**

(84) Benannte Vertragsstaaten:
**AT BE CH DE DK ES FR GB IT LI NL**

(71) Anmelder: **F. HOFFMANN-LA ROCHE AG**
**Postfach 3255**
**CH-4002 Basel (CH)**

(72) Erfinder: **Veits, Joachim**
**14 Eggbergstrasse**
**D-79618 Rheinfelden (DE)**

(74) Vertreter: **Kellenberger, Marcus, Dr. et al**
**Grenzacherstrasse 124**
**Postfach 3255**
**CH-4002 Basel (CH)**

(54) **Herstellung von 2-Keto-L-gulonsäureestern.**

(57) Ein Verfahren zur Herstellung von 2-Keto-L-gulonsäure-methyl- oder -äthylester durch Veresterung von 2-Keto-L-gulonsäure mit Methanol bzw. Aethanol in Gegenwart eines sauren Katalysators besteht darin, dass man die Veresterung kontinuierlich, in Gegenwart eines sauren Ionenaustauschers, im Temperaturbereich zwischen Raumtemperatur und etwa 80°C und bei mittleren Verweilzeiten zwischen etwa 10 und etwa 120 Minuten und bei Leerrohrgeschwindigkeiten von etwa 0,5 m/h bis etwa 7,5 m/h durchführt. Die Veresterung erfolgt vorteilhaft unter leichtem Ueberdruck. Die so hergestellten Ester stellen wichtige Zwischenprodukte für die Synthese von Vitamin C dar, und das jeweilige Veresterungsprodukt kann unmittelbar in Vitamin C durch Laktonisierung übergeführt werden.

EP 0 671 405 A1

Die vorliegende Erfindung betrifft ein neues Verfahren zur Herstellung von 2-Keto-L-gulonsäureestern. Diese Ester stellen bekanntlich wichtige Zwischenprodukte für die Synthese von Ascorbinsäure (Vitamin C) dar.

Die Veresterung von 2-Keto-L-gulonsäure mit einem niederen Alkanol, insbesondere Methanol, ist aus zahlreichen Publikationen bekannt. Eine solche Veresterung erfolgt üblicherweise in Gegenwart eines sauren Katalysators, z.B. Salzsäure, Schwefelsäure oder p-Toluolsulfonsäure. Nach der Veresterung zum entsprechenden Niederalkylester kann dieser einer sogenannten alkalischen Umlagerung (oder Laktonisierung) zur Ascorbinsäure oder zu einem Salz davon, üblicherweise dem Natrium- oder Kaliumsalz, unterworfen werden.

In der Fachliteratur finden sich gelegentlich Hinweise auf die Veresterung von 2-Keto-L-gulonsäure, die in Gegenwart eines Ionenaustauschers als saurem Katalysator erfolgt. Es bleiben jedoch meistens Angaben zu Ausbeuten, Umsätzen usw. aus, oder die berichteten Resultate, z.B. in bezug auf Ausbeuten und Kristallqualität, sind unbefriedigend. Zudem hat sich bisher ein Verfahren zur kontinuierlichen Veresterung von 2-Keto-L-gulonsäure nicht durchgesetzt bzw. wurde nicht veröffentlicht.

Grundsätzlich sollte ein Veresterungsverfahren zur Herstellung eines 2-Keto-L-gulonsäureesters, insbesondere des Methyl- oder Aethylesters, einfach bei der Durchführung, schonend und mit hohem Umsatz verlaufen, wobei eine kontinuierliche Verfahrensweise anzustreben ist.

Es wurde nun ein solches Veresterungsverfahren gefunden, das die genannten Anforderungen in hohem Mass erfüllt und gegenüber herkömmlichen Verfahren Vorteile aufweist. Das erfindungsgemässe Verfahren zur Veresterung von 2-Keto-L-gulonsäure mit Methanol oder Aethanol zu deren Methyl- bzw. Aethylester ist dadurch gekennzeichnet, dass man die Veresterung kontinuierlich, in Gegenwart eines sauren Ionenaustauschers, im Temperaturbereich zwischen Raumtemperatur und etwa 80°C und bei mittleren Verweilzeiten zwischen etwa 10 und etwa 120 Minuten und bei Leerrohrgeschwindigkeiten von etwa 0,5 m/h bis etwa 7,5 m/h durchführt (h = Stunde).

In der obigen Definition des Erfindungsgegenstandes umfasst der Begriff "saurer Ionenaustauscher" diejenigen sauren Ionenaustauscher, die typischerweise durch die kommerziell erhältlichen Duolite® C 20, C 26, C 264 und C 265 (alle Rohm & Haas); Amberlite® 18 wet und IRA 120 (ebenfalls Rohm & Haas); Amberlyst® 15 (Rohm & Haas); Lewatit® S 100 und SP 112 (Bayer); sowie Dowex® Ionenaustauscher I- (Dow) vertreten sind. Von diesen sauren Ionenaustauschern eignet sich zum Zweck der vorliegenden Erfindung Amberlyst® 15 am besten. Grundsätzlich kommen allerdings weitere saure Ionenaustauscher in Frage.

Der ebenfalls in der Erfindungsdefinition erscheinende Begriff "Leerrohrgeschwindigkeit" bezeichnet die Fliessgeschwindigkeit, mit der eine flüssige Reaktionsmasse durch einen rohr-(oder kolonnen-)artigen, mit Katalysator gefüllten Reaktor fliesst, und die wie nachstehend aufgeführt vom Durchsatzvolumen und vom Rohrquerschnitt des Reaktors abhängt. Diese Geschwindigkeit hängt im weiteren Sinne mit der mittleren effektiven Geschwindigkeit zusammen. Mathematisch ausgedrückt gilt:

$$\text{Leerrohrgeschwindigkeit } (V_{Leerrohr}) = \frac{\text{Durchsatzvolumen } [cm^3 \text{ oder } m^3]}{\text{Rohrquerschnitt } [cm^2 \text{ bzw. } m^2] \text{ x Zeit } [h]}$$

wobei $V_{Leerrohr}$ in der Dimension cm/h bzw. m/h ausgedrückt ist.

Wie oben erwähnt, hängt mit der Leerrohrgeschwindigkeit $V_{Leerrohr}$ die sogenannte mittlere effektive Geschwindigkeit $V_{effektiv}$ unmittelbar zusammen, und zwar gemäss der Gleichung:

$$V_{effektiv} = \frac{V_{Leerrohr}}{\varepsilon}$$

worin

$$\varepsilon \text{ (der Lückengrad)} = \frac{V_R - V_K}{V_R}$$

EP 0 671 405 A1

($V_R$ = Reaktorvolumen; $V_K$ = Katalysatorvolumen)

Diese Begriffe sind u.a. im Lehrbuch "Thermische Verfahrenstechnik" von A. Mersmann, S. 99ff, Springer-Verlag Berlin Heidelberg, New York 1980 näher erläutert.

Im wesentlichen wird die Veresterung so durchgeführt, dass man eine Lösung der 2-Keto-L-gulonsäure im Methanol oder Aethanol über den im Reaktor befindlichen sauren Ionenaustauscher fliessen lässt. Die Veresterung wird im allgemeinen im Temperaturbereich zwischen Raumtemperatur und etwa 80°C, vorzugsweise bei Temperaturen zwischen etwa 55°C und etwa 65°C, und bei mittleren Verweilzeiten zwischen etwa 10 und etwa 120 Minuten durchgeführt. Zudem erfolgt die Veresterung zweckmässigerweise unter leichtem Ueberdruck bis maximal 4 bar, und zwar unabhängig von der Veresterungstemperatur. Auf den Gesamtumsatz betrachtet, besteht eine leichte Abhängigkeit von der Veresterungstemperatur und der Konzentration der 2-Keto-L-gulonsäure im Methanol bzw. Aethanol: im allgemeinen gilt es, dass höhere Temperaturen kürzere mittlere Verweilzeiten erfordern. Bei Konzentrationen im Bereich von etwa 8 bis etwa 15 Gewichtsprozent 2-Keto-L-gulonsäure in Methanol verläuft die Veresterung (nahezu) ideal bei etwa 60°C und Verweilzeiten von weniger als 20 Minuten. Unter solchen Bedingungen beträgt der Restgehalt an 2-Keto-L-gulonsäure normalerweise höchstens 1%.

Im allgemeinen wird die erfindungsgemässe Veresterung mit Konzentrationen an 2-Keto-L-gulonsäure im Methanol oder Aethanol im Bereich von etwa 2,5 bis etwa 15 Gewichtsprozent durchgeführt. Im Falle der Verwendung von Methanol liegt die Konzentration zweckmässigerweise im Bereich von 8 bis 15 Gewichtsprozent (G/G-%), was einer in Gewicht/Volumen-Prozent (G/V-%) ausgedrückten Konzentration von 7 bis 14 G/V-% entspricht, und zwar bezogen auf wasserfreie 2-Keto-L-gulonsäure. Die zu veresternde Säure kann als wasserfreie oder hydratisierte 2-Keto-L-gulonsäure(insbesondere das Monohydrat) eingesetzt werden. Die wasserfreie Form ist jedoch bevorzugt. Es hat sich herausgestellt, dass zumindest im Konzentrationsbereich 8 bis 12,2 G/G-% der Säure in Methanol die Veresterung praktisch unabhängig von der Konzentration abläuft: der Umsatzverlauf ist in diesem Konzentrationsbereich nahezu gleich (Reaktion nullter bzw. pseudonullter Ordnung bezüglich der 2-Keto-L-gulonsäure).

Wie oben erwähnt, stellt unter anderem die Leerrohrgeschwindigkeit ein wesentliches Erfindungsmerkmal dar. Der diesbezügliche Bereich von etwa 0,5 m/h bis etwa 7,5 m/h entspricht einer mittleren effektiven Geschwindigkeit von etwa 0,9 bis etwa 14,5 m/h.

Ein typischer Anlageaufbau für das erfindungsgemässe Veresterungsverfahren wird wie folgt schematisch dargestellt, wobei die angegebenen Dimensionen eher für eine Anlage im kleinen Massstab gelten. Im kommerziellen Massstab gelten selbstverständlich entsprechend grössere Dimensionen.

Das erfindungsgemässe Verfahren weist mehrere Vorteile auf, und zwar:
- die Arbeitsweise ist kontinuierlich und deshalb effizient bei der Durchführung;
- die Verweilzeiten sind im Vergleich zu bisherigen Veresterungen von 2-Keto-L-gulonsäure mit Methanol oder Aethanol relativ kurz;
- die praktische (apparative) Durchführung des Verfahrens ist einfach und wirtschaftlich, zumal beispielsweise sowohl eine Wasserentfernung zwecks Gleichgewichtsverschiebung wie auch hohe Reaktionstemperaturen nicht erforderlich sind;
- die Korrosion einer metallhaltigen Verfahrensanlage wird vermieden, da der zur Durchführung der Veresterung verwendete Katalysator keine Mineralsäure, z.B. Salzsäure, Schwefelsäure usw., ist;
- die Gleichgewichtsumsätze sind hoch, wobei das Produkt je nach spezifischen Reaktionsbedingungen einen Restgehalt an nicht umgesetzter 2-Keto-L-gulonsäure von nur etwa 0,5 bis 1,5 Gewichtsprozent aufweist; und
- das Veresterungsprodukt kann unmittelbar, d.h. ohne Isolierung des 2-Keto-L-gulonsäure-methyl- bzw. -äthylesters, in den Laktonisierungsschritt zur Herstellung der gewünschten Ascorbinsäure, z.B. unter alkalischen Bedingungen (unter Verwendung von beispielsweise Natriumbicarbonat, Natriumhydroxid, Natriummethoxid oder Trihexylamin als Base), eingesetzt werden.

Das erfindungsgemässe Verfahren umfasst auch verschiedene Durchführungsvarianten der oben beschriebenen, relativ einfachen Arbeitsweise, und zwar mit dem Ziel, den ohnehin sehr hohen Gleichgewichtsumsatz noch zu steigern. Das Reaktionsgut kann beispielsweise mehrere (zwei oder mehr) hintereinandergeschaltete Reaktoren bis zum Erreichen des gewünschten (besonders hohen) Umsatzes durchlaufen. Ferner kann nach Durchlauf eines Reaktors eine partielle Zwischeneindampfung, d.h. teilweise Wasserentfernung mit dem gleichen Alkanol, z.B. Methanol, erfolgen und das Reaktionsgut anschliessend eine nachgeschaltete Veresterungssäule durchlaufen, und zwar mit oder ohne Zusatz von frischem Methanol bzw. Aethanol.

Das erfindungsgemässe Verfahren wird anhand der nachfolgenden Beispiele veranschaulicht, wobei jeweils auf den oben erwähnten schematisch dargestellten, typischen Anlageaufbau verwiesen wird.

3

Beispiel 1

In ein 10 l Glasgefäss werden in 2 Portionen jeweils 917,45 g 2-Kete-L-gulonsäure (nachfolgend "2-KLGS"; 98,1%ige Qualität) und 10 l Methanol eingetragen und solange gerührt, bis sich eine homogene Lösung gebildet hat. Ueber ein GELMAN-Membranfilter 0,2 μm wird das Gemisch klarfiltriert und die Lösung portionsweise in das Vorratsgefäss (3) transferiert. Beheizt wird das Vorratsgefäss gegebenenfalls unter Einsatz eines Thermostaten (1) bei einer Temperatur von 35°C.

Dann wird das Gemisch über ein Bodenventil entnommen und mittels einer LEWA M8 Membranpumpe (5) mit einer Fördergeschwindigkeit von 800 ml/h zunächst über eine kleine, mit Amberlyst® 15 gefüllte Vorsäule und danach durch das auf 60-62°C beheizte, im Doppelmantelrohr (6) befindliche Austauscherbett gepumpt. Die gefüllte Vorsäule hat eine Schutzfunktion, indem sie Metallspuren, z.B. Eisen, Kupfer, Zink usw., abfängt. Die Beheizung der Säule erfolgt bei einer Temperatur von 63°C mittels eines Thermostaten (2). Zur Vermeidung von Siedeeffekten und Gasblasenbildung wird die Anlage mit einem am Säulenausgang installierten Druckhalteventil (8) auf 0,4-0,6 bar Ueberdruck (7) eingestellt. Zur Umsatzkontrolle werden in 2-stündigen Intervallen Muster abgenommen und jeweils per HPLC der restliche 2-KLGS-Gehalt ermittelt. Parallel hierzu wird eine Kontrolle der Fördermenge vorgenommen, und zwar unter Verwendung eines graduierten 50 ml Schüttelzylinders. Unter den angegebenen Bedingungen beträgt die Leerrohrgeschwindigkeit 2,86 m/h und die mittlere effektive Geschwindigkeit 5,53 m/h. Die mittlere Verweilzeit liegt bei ca. 14,6 Minuten.

Nach erfolgtem Gemischdurchsatz werden zur Spülung der Anlage weitere 3 l Methanol durchgepumpt.

Zur Isolierung des so hergestellten 2-Keto-L-gulonsäure-methylesters (nachfolgend "Me-2-KLGS") wird die gesammelte Veresterungslösung (9) in einem BÜCHI Rotavapor Typ R 152 bei einer Badtemperatur von 50°C und einem Druck von 200 mbar zu einem Kristallbrei (ca. 67 G/G-% Me-2-KLGS) eingeengt und ca. 4 Stunden zur Nachkristallisation bei 4°C gekühlt. Das Kristallisat wird daraufhin über eine Glassinternutsche abgesaugt und zweimal mit jeweils 500 ml Methanol (-10°C) nachgewaschen. Die Trocknung erfolgt bei 50°C und 10-15 mbar innerhalb von 12 Stunden. Auswaage: 1700-1800 g 1. Kristallisat Me-2-KLGS, Qualität ≧ 99,5%.

Zur Isolierung des 2. Kristallisates wird die Mutterlauge ebenfalls bei 50°C und 200 mbar zu einem Kristallbrei eingeengt (ca. 43 G/G-% Me-2-KLGS-Anteil), über 4 Stunden bei 4°C gekühlt und das Kristallisat über eine Glassinternutsche abgesaugt. Dann wird das Produkt zweimal mit jeweils 130 ml Methanol (-10°C) nachgewaschen und bei 50°C unter Vakuum etwa 12 Stunden getrocknet. Auswaage: ca. 100 g 2. Kristallisat Me-2-KLGS, Qualität > 98,0%.

Der Gesamtumsatz zu Me-2-KLGS beläuft sich ausgehend von 2-KLGS und ohne die als Nebenprodukt gebildete Ascorbinsäure auf 97,5-97,9% (mittels HPLC quantifiziert), und aus der praktisch farblosen Veresterungslösung werden 95,7-95,9% der Theorie an Me-2-KLGS als farblose Kristalle isoliert (1. und 2. Kristallisat, Qualität bereits berücksichtigt).

Eine Kristallisolierung muss nicht erfolgen, da die Lösung erwünschtenfalls unmittelbar weiter verwendet werden kann, und zwar zur Herstellung von Ascorbinsäure, z.B. durch alkalische Umlagerung (Laktonisierung).

Beispiel 2

Wie in Beispiel 1 beschrieben wird eine 10,2 G/G-%-ige Lösung von 2-KLGS in Methanol bei einer Temperatur von 30°C und mit einer Leerrohrgeschwindigkeit von 0,63 m/h und einer mittleren Verweilzeit von ca. 116 Minuten über die Säule gepumpt. Es erfolgt Umsatz auf einen Rest-2-KLGS-Gehalt von ca. 5,1%. Nochmaliges Durchlaufen der Lösung unter gleichen Bedingungen, d.h. ohne zwischenzeitliche, partielle Entfernung von Methanol/Wasser, führt zu einem Rest-2-KLGS-Gehalt von ca. 1,6%.

Beispiel 3

Wie in Beispiel 1 beschrieben, wird eine 10,2 G/G-%ige Lösung von 2-KLGS in Methanol bei einer Temperatur von 60-62°C und mit einer Leerrohrgeschwindigkeit von ca. 5,7 m/h (entspricht einer mittleren effektiven Geschwindigkeit von ca. 11 m/h) und einer Verweilzeit von ca. 6,5 Min. über die Säule gepumpt. Es erfolgt Umsatz auf einen Rest-2-KLGS-Gehalt von ca. 7,3-9,1%. Nochmaliges Durchlaufen der Lösung unter gleichen Bedingungen führt zu einem Rest-2-KLGS-Gehalt von ca. 1,3-1,7%. Die Gesamtverweilzeit beträgt danach 2x ca. 6,5 Min., also ca. 13 Min.

Beispiel 4

Analog der in Beispiel 1 beschriebenen Arbeitsweise werden mehrere 2-KLGS-Veresterungen durchgeführt, und zwar unter diversen Bedingungen. Die diesbezüglichen Daten sind in der nachfolgenden Tabelle 1 zusammengefasst:

Tabelle 1

| Typische Beispiele / Resultate der Veresterung bei 60-62 °C (idealer Bereich) | | | | |
|---|---|---|---|---|
| Konzentration [G/G-%] | Verweilzeit [Min] | Leerrohrgeschwindigkeit [m/h] | Mittlere effective Geschwindigkeit [m/h] | Rest- 2-KLGS [%] |
| 8,2 | 29,0 | 1,27 | 2,45 | 0,42 |
| | 19,5 | 1,91 | 3,69 | 0,42 |
| | 14,5 | 2,54 | 4,91 | 1,40 |
| 10,2 | 23,5 | 1,59 | 3,07 | 0,63 |
| | 16,7 | 2,23 | 4,31 | 0,65 |
| | 14,6 | 2,86 | 5,53 | 0,80 |
| | 13,0 | 3,50 | 6,77 | 0,96 |
| 12,2 | 29,0 | 1,27 | 2,45 | 0,91 |
| | 19,5 | 1,91 | 3,69 | 1,00 |
| | 14,5 | 2,54 | 4,91 | 1,32 |
| 15,0 | 29,0 | 1,27 | 2,45 | 0,90 |
| | 19,5 | 1,91 | 3,69 | 1,10 |
| | 14,5 | 2,54 | 4,91 | 1,35 |

Beispiel 5

Analog der in Beispiel 1 beschriebenen Arbeitsweise werden weitere 2-KLGS-Veresterungen durchgeführt (Versuche 1-6). Die mittlere Verweilzeit beläuft sich hierbei auf ca. 14,6 Min., die Leerrohrgeschwindigkeit auf ca. 2,86 m/h. Die diesbezüglichen restlichen Daten sind in der nachfolgenden Tabelle 2 zusammengefasst:

**Tabelle 2** - Einsatz: 1800 g 2-KLGS ergibt 1929,6g Me-2-KLGS gemäss der Theorie

| Bezeichnung (Vs = Versuch) | | Vs 1 | Vs 2 | Vs 3 | Vs 4 | Vs 5 | Vs 6 |
|---|---|---|---|---|---|---|---|
| 1. Rest-2-KLGS [1] | [%] | 0,82 | 0,86 | 0,70 | 0,85 | 0,85 | 0,82 |
| 2. 1. Kristallisat Me-2-KLGS | | | | | | | |
| Auswaage | [g] | 1678,9 | 1731,0 | 1752,8 | 1793,0 | 1818,6 | 1754,9 |
| Gehalt | [%] | 99,5 | 99,8 | 99,6 | 98,5 | 99,4 | 99,4 |
| Menge (100%) | [g] | 1670,1 | 1727,5 | 1745,8 | 1766,1 | 1807,7 | 1743,4 |
| Anteil der Theorie | [%] | 86,6 | 89,5 | 90,5 | 91,5 | 93,7 | 90,4 |
| 3. 2. Kristallisat Me-2-KLGS | | | | | | | |
| Auswaage | [g] | 178,0 | 118,4 | 101,7 | 79,6 | 57,1 | 107,0 |
| Gehalt | [%] | 98,0 | 98,8 | 96,8 | 94,3 | 94,0 | 96,4 |
| Menge (100%) | [g] | 174,4 | 117,0 | 98,4 | 75,1 | 53,7 | 103,7 |
| Anteil der Theorie | [%] | 9,0 | 6,1 | 5,1 | 3,4 | 2,8 | 5,4 |
| 4. Mutterlauge 2 | | | | | | | |
| Me-2-KLGS-Anteil | [g] | 54,1 | 40,5 | 44,1 | 27,4 | 40,4 | 41,3 |
| Anteil der Theorie | [%] | 2,8 | 2,1 | 2,3 | 1,4 | 2,1 | 2,1 |
| 5. Bilanzierung | | | | | | | |
| Me-2-KLGS isoliert, gesamt (100%) | [g] | 1844,5 | 1844,5 | 1844,2 | 1841,2 | 1861,4 | 1847,2 |
| Anteil der Theorie | [%] | 95,6 | 95,6 | 95,6 | 95,4 | 96,5 | 95,7 |
| Menge Me-2-KLGS, gesamt | [g] | 1898,6 | 1885,0 | 1888,3 | 1868,3 | 1901,8 | 1888,5 |
| Anteil der Theorie, gesamt | [%] | 98,4 | 97,7 | 97,9 | 96,8 | 98,6 | 97,9 |

[1] Diese Werte wurden durch Normierung aus den Peakflächen Me-2-KLGS, Ascorbinsäure und 2-KLGS ermittelt.

EP 0 671 405 A1

## Apparaturaufbau für 2-KLGS-Veresterung

1  Thermostat, Typ JULABO VC5, +35 °C
2  Thermostat, Typ JULABO SC17 B, +63 °C
3  Weithalsdoppelmantelkolben, Typ HWS
   4 Liter Inhalt
4  Filter, Typ SARTORIUS
5  Membranpumpe, Typ LEWA M8
6  Doppelmantelrohr, V4A
   Innendurchmesser:    20 mm
   Länge:      120 cm
   Füllmenge A 15:      231 g
   Lückenvolumen:            195 ml
7  Manometer
8  Druckhalteventil, Typ TESCOM
9  Vorlage, 20 Liter-Kanister
10 Intensivkühler, Länge 28 cm
11 Vorsäule (A 15)

Ionenaustauscher:
Amberlyst 15 (A 15), ROHM & HAAS Co.

## Patentansprüche

1.  Verfahren zur Herstellung von 2-Keto-L-gulonsäure-methyl- oder äthylester durch Veresterung von 2-Keto-L-gulonsäure mit Methanol bzw. Aethanol in Gegenwart eines sauren Katalysators, dadurch gekennzeichnet, dass man die Veresterung kontinuierlich, in Gegenwart eines sauren Ionenaustauschers, im Temperaturbereich zwischen Raumtemperatur und etwa 80 °C und bei mittleren Verweilzeiten zwischen etwa 10 und etwa 120 Minuten und bei Leerrohrgeschwindigkeiten zwischen etwa 0,5 m/h und etwa 7,5 m/h durchführt.

2. Verfahren nach Anspruch 1, dadurch gekennzeichnet, dass man mit Methanol verestert.

3. Verfahren nach Anspruch 1 oder 2, dadurch gekennzeichnet, dass der saure Ionenaustauscher Amberlyst® 15 ist.

4. Verfahren nach einem der Ansprüche 1 bis 3, dadurch gekennzeichnet, dass die Veresterung bei Temperaturen zwischen etwa 55°C und etwa 65°C durchgeführt wird.

5. Verfahren nach einem der Ansprüche 1 bis 4, dadurch gekennzeichnet, dass die Veresterung unter leichtem Ueberdruck bis maximal 4 bar erfolgt.

6. Verfahren nach einem der Ansprüche 2 bis 5, dadurch gekennzeichnet, dass die Konzentration an 2-Keto-L-gulonsäure in Methanol im Bereich von 8 bis 15 Gewichtsprozent ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | DE-A-17 68 104 (WACKER CHEMIE GMBH) 2.März 1972 <br> * das ganze Dokument * <br> --- | 1-6 | C07H7/027 |
| A | EP-A-0 403 993 (ENCO ENGINEERING CHUR AG) 27.Dezember 1990 <br> *Beispiele* <br> --- | 1-6 | |
| A | EP-A-0 138 436 (TAKEDA CHEMICAL INDUSTRIES LTD) 24.April 1985 <br> * Beispiele 15,17,19 * <br> --- | 1-6 | |
| Y | J. PRAKT. CHEM., <br> Bd. 1, 1955 <br> Seiten 153-156, <br> G. DREFAHL 'Ester der 2-Keto-L-gulonsäure, der Lävulinsäure und Schleimsäure' <br> * das ganze Dokument * <br> --- | 1-6 | |
| A | CHEMICAL ABSTRACTS, vol. 119, no. 25, 1993 <br> Columbus, Ohio, US; <br> abstract no. 271553, <br> T.A. MELENTYEVA ET AL. 'Methyl ester of 2-keto-1-gulonic acid' <br> Seite 1042; Spalte r; <br> * Zusammenfassung * <br> & KHIM.-FARM. ZH., <br> Bd. 26, 1992 <br> Seiten 112-3, <br> ----- | 1-6 | **RECHERCHIERTE SACHGEBIETE (Int.Cl.6)** <br><br> C07H |

Der vorliegende Recherchenbericht wurde für alle Patentansprüche erstellt

| Recherchenort | Abschlußdatum der Recherche | Prüfer |
|---|---|---|
| MÜNCHEN | 26.Juni 1995 | Bardili, W |

EPO FORM 1503 03.82 (P04C03)